# EUROPÄISCHE PATENTANMELDUNG

(11) **EP 3 047 820 A1**
(43) Veröffentlichungstag der Anmeldung: **27.07.2016**
(21) Anmeldenummer: 15186801.5
(22) Anmeldetag: 25.09.2015
(51) Int. Cl.: A61F 2/966, A61F 2/95

(54) **SYSTEM ZUM EINFÜHREN UND FREISETZEN EINES SELBSTEXPANDIERENDEN STENTS UND VERFAHREN HIERFÜR**

(30) Priorität: 21.01.2015 DE 102015200963
(71) Anmelder: Joline GmbH & Co. KG, 72379 Hechingen (DE)
(72) Erfinder: EISOLD, Gerd, 72415 Grosselfingen (DE); MAIER, Jan, 72116 Mössingen (DE)
(74) Vertreter: DREISS Patentanwälte PartG mbB

(57) **Zusammenfassung**

System zum Einführen eines komprimierten, selbstexpandierbaren Stents in insbesondere ein Blutgefäß und zur Freisetzung des Stents in dem insbesondere einen Blutgefäß, umfassend einen Katheter mit einem Innenschlauch, einem Mittelschlauch und einem Außenschlauch, wobei sich der Mittelschlauch durch den Außenschlauch erstreckt und sich der Innenschlauch durch den Mittelschlauch erstreckt, wobei der komprimierte Stent in einem distalen Endabschnitt des Katheters radial um den Innenschlauch verlaufend zwischen dem Innenschlauch und dem Außenschlauch radial komprimiert angeordnet ist und der Mittelschlauch distal am proximalen Ende des Stents endet, wobei zur Freisetzung des Stents der Außenschlauch in proximaler Richtung und der Mittelschlauch in distaler Richtung derart verschiebbar sind, dass das distale Ende des Stents während der Freisetzung seine Bestimmungsposition beibehält.

## Beschreibung

Die Erfindung betrifft ein System zum Einführen eines komprimierten, selbstexpandierbaren Stents in insbesondere ein Blutgefäß und zur Freisetzung des Stents in dem insbesondere einen Blutgefäß. Die Erfindung betrifft auch einen dazugehörigen Katheter, eine Antriebseinheit und ein Verfahren.

Aus dem Stand der Technik sind unterschiedliche Arten von Stents vorbekannt. Insbesondere sind lasergeschnittene Stents bereits seit längerem bekannt. Diese passen sich einem Lumen allerdings nur bedingt an und es hat sich herausgestellt, dass diese in einem Lumen nach einigen Jahren brechen. Deswegen wurden Flechtstents entwickelt, welche nicht nur flexibler sind und leichter in ein Lumen einsetzbar sind, sondern auch noch nach Jahren des Einsatzes in einem Lumen nicht brechen und kostengünstiger herstellbar sind. Derartige Flechtstents dehnen sich bei der Freisetzung in einem Lumen in radialer Richtung aus und weiten somit ein Lumen auf und halten das Lumen aufgeweitet. Allerdings ändert sich bei der Freisetzung die Position der Stents insbesondere auf Grund der Änderung der Länge und des Durchmessers.

Aus dem Stand der Technik sind weiterhin verschiedene Einführsysteme für insbesondere geflochtene, selbstexpandierbare Stents vorbekannt. In der Regel handelt es sich dabei um Kathetersysteme, mithilfe derer der Stent an seinen Einsatzort geschoben und daraufhin dadurch expandiert wird, dass der Stent aus dem Katheter freigesetzt wird. Mit solchen Einführsystemen werden Gefäßstents in Blutgefäße implantiert, welche aufgrund einer Krankheit oder ähnlichem verletzt oder in ihrem Lumen verschlossen oder verengt sind, wodurch die Gefäße in ihrer Funktion stark beeinträchtigt sind. Aus dem Stand der Technik sind verschiedene

implantierbare Stents vorbekannt, die Blutgefäße, beispielsweise Arterien nach ihrer Implantation offenhalten. Derartige Stents haben regelmäßig einen röhrenförmigen Körper, der in das Blutgefäß eingeführt und an der entsprechenden Stelle fixiert wird, um das Lumen des Gefäßes aufzuweiten oder aufrechtzuerhalten.
Zur Implantation wird der Stent in der Regel radial zusammengedrückt, sodass sich seine Querschnittsfläche deutlich verringert, wobei sich seine Länge deutlich verlängert, sodass er relativ einfach in das Gefäß eingeführt werden kann. Handelt es sich bei dem Stent um einen selbstexpandierenden Stent, so expandiert dieser sich aufgrund seiner Elastizität bzw. Federwirkung bei einer Freisetzung selbst wieder in seine ursprüngliche Form und spannt dabei seine Mantelfläche auf, sodass diese sich in dem Blutgefäß anlegt beziehungsweise das Lumen des Blutgefäßes aufweitet oder aufrechterhält.

In der Regel werden die radial komprimierten Stents mithilfe von Kathetern in das Blutgefäß eingeführt und einer Bestimmungsposition zugeführt. Die richtige Lage des Stents kann dabei insbesondere über Röntgenmarker kontrolliert werden.

Aus dem Stand der Technik sind zudem Katheder vorbekannt, in denen die komprimierten Stents in einem hülsenartigen Schlauch angeordnet sind, der aufgrund seiner elastischen Eigenschaften den Stent radial nach innen drückt. Das proximale Ende des Stents wird dabei axial von einem Anschlagrohr gehalten. Der hülsenartige Schlauch, die sogenannte Rückzugshülle, wird nach dem Positionieren des Stents im Gefäß zurückgezogen, wobei das Anschlagrohr in Anlage mit dem Stent ist. Das proximale Ende des Stents wird folglich während der Freisetzung unverändert beibehalten. Während der Freisetzung wird die Rückzugshülle, welche auch das Anschlagrohr umgibt, von dem Stent abgezogen, welcher sich dabei expandieren und in dem Blutgefäß anlegen kann. Bei einer derartigen Platzierung des Stents hat es sich herausgestellt, dass es unmöglich ist, diesen exakt zu positionieren, da sich die Länge des Stents während der Freisetzung bedingt durch die Selbstexpansion verringert und sich der Stent somit verlagert.

Mit herkömmlichen Einführsystemen insbesondere für geflochtene Stents ist somit auch hocherfahrenen Anwendern regelmäßig eine millimetergenaue Positionierung des Stents nicht möglich. Deswegen besteht der Bedarf nach einem Einführsystem für insbesondere geflochtene Stents, mit Hilfe dessen Stents exakt positioniert werden können und damit nach der Freisetzung ihre Bestimmungsposition einnehmen. Der folgenden Erfindung liegt die Aufgabe zugrunde, den genannten Nachteilen des Standes der Technik Abhilfe zu schaffen, insbesondere eine punktgenaue Platzierung des Stents mit optimaler Expansion zu ermöglichen.

Diese Aufgabe wird mit einem System mit den Merkmalen des Anspruchs 1 gelöst. Das System umfasst folglich einen Katheter mit einem Innenschlauch, einem Mittelschlauch und einem Außenschlauch, wobei sich der Mittelschlauch durch den Außenschlauch erstreckt und sich der Innenschlauch durch den Mittelschlauch erstreckt. Dabei ist der komprimierte Stent in einem distalen Endabschnitt des Katheters radial um den Innenschlauch verlaufend zwischen dem Innenschlauch und dem Außenschlauch radial komprimiert angeordnet. Weiterhin endet der Mittelschlauch distal am proximalen Ende des Stents. Zur Freisetzung des Stents sind der Außenschlauch in proximaler Richtung und der Mittelschlauch in distaler Richtung derart verschiebbar, dass das distale Ende des Stents während der Freisetzung seine Bestimmungsposition beibehält. Die Bestimmungsposition kann dabei innerhalb eines vorgegebenen Bereichs, insbesondere zwischen 0 und 5 mm, liegen.

Bei dem selbstexpandierbaren Stent kann es sich dabei insbesondere um einen selbstexpandierbaren, geflochtenen Stent oder um einen mittels anderer Verfahren hergestellten Stent aus Metalldraht oder Kunststofffaden handeln. Insbesondere bei geflochtenen Stents besteht die Problematik, dass sich diese bei der Freisetzung sehr stark ausdehnen und damit nach der Freisetzung nicht ihre Bestimmungsposition einnehmen. Eine exakte Positionierung ist aus medizinischer Sicht jedoch unbedingt erwünscht bzw. notwendig.

Dadurch, dass vorgesehen ist, dass der Katheter aus drei Schläuchen besteht, wobei zur Freisetzung des Stents der Außenschlauch in proximaler Richtung und der Mittelschlauch in distaler Richtung verschiebbar sind, kann erreicht werden, dass sich die Bestimmungsposition des distalen Endes des Stents während der Freisetzung nicht oder nur geringfügig ändert. Das Führungselement in Gestalt des Innenschlauchs bleibt dabei unverändert, während der Mittelschlauch und der Außenschlauch eine Relativbewegung zum Innenschlauch durchführen können. Die Freisetzung des Stents erfolgt also dadurch, dass der Außenschlauch in proximaler Richtung vom komprimierten Stent abgezogen wird, während der Mittelschlauch in distaler Richtung das proximale Ende des komprimierten Stents nach vorne, also in distale Richtung schiebt. Die Relativbewegung des Mittelschlauchs und des Außenschlauchs zum Innenschlauch kann dabei synchron oder abwechselnd erfolgen.

Insgesamt wird mittels des erfindungsgemäßen Systems erreicht, dass nach der Freisetzung des Stents, nicht nur das distale Ende des Stents, sondern auch das proximale Ende des Stents seine Bestimmungsposition einnimmt und damit der gesamte Stent nach der Freisetzung seine Bestimmungsposition einnimmt. Folglich nimmt der Stent nach der Freisetzung seine Solllänge und damit seine Gesamtbestimmungsposition ein, wohingegen gemäß dem Stand der Technik entweder die distale und/oder proximale Bestimmungsposition und/oder die Solllänge nicht eingenommen wird/werden.

Um die entsprechenden Relativbewegungen des Außenschlauchs und des Mittelschlauchs relativ zum Innenschlauch auszuführen, ist vorteilhafterweise im proximalen Bereich des Katheters eine Antriebseinheit vorgesehen, wobei die Antriebseinheit mit einem Bewegungsabschnitt des Außenschlauchs und mit einem Bewegungsabschnitt des Mittelschlauchs zusammenwirkt. Dabei wird der äußere Schlauch in proximaler Richtung bewegt, während der Mittelschlauch in distaler Richtung bewegt wird, wobei die Bewegung des Außenschlauchs beziehungsweise des Mittelschlauchs mittels der Antriebseinheit synchron oder abwechselnd erfolgen kann.

Vorzugsweise umfasst die Antriebseinheit dabei ein Schiebelement, wobei das Schiebeelement in eine erste Schiebeelementbewegungsrichtung und eine zweite Schiebeelementbewegungsrichtung axial, also insbesondere in Längsrichtung der Schläuche bzw. des Katheters hin und her bewegbar ist. Dabei kann am Schiebeelement ein erstes Mitnahmemittel und ein zweites Mitnahmemittel angeordnet sein, wobei das erste Mitnahmemittel mit dem Bewegungsabschnitt des Außenschlauchs zusammenwirkt und wobei das zweite Mitnahmemittel mit dem Bewegungsabschnitt des Mittelschlauchs zusammenwirkt. Damit kann eine besonders einfach aufgebaute Antriebseinheit bereitgestellt werden. Eine Bewegung des Mittelschlauchs und des Außenschlauchs kann über eine Schiebebewegung des Schiebeelements bereitgestellt werden. Weiterhin kann eine Bedienperson, die die Antriebseinheit bedient, die Freisetzung des Stents sehr präzise steuern, indem händisch oder automatisiert das Schiebeelement bewegt wird. Bei den Mitnahmemitteln kann es sich dabei um Eingriffzungen handeln. Diese leiten eine Kraft mittelbar oder unmittelbar am Bewegungsabschnitt des Mittelschlauchs beziehungsweise des Außenschlauchs ein.

Alternativ ist denkbar, dass die Antriebseinheit ein Drehrad umfasst, wobei das Drehrad einen ersten Mitnahmeabschnitt aufweist, welcher in den Außenschlauch eingreift, und wobei das Drehrad einen zweiten Mitnahmeabschnitt aufweist, welcher in den Mittelschlauch eingreift. Somit kann durch Drehen des Drehrades eine Bewegung des Außenschlauchs und des Mittelschlauchs erzielt werden. Dabei ist andererseits auch denkbar, dass die Antriebseinheit ein erstes Drehrad zum Antreiben des Außenschlauchs und ein zweites Drehrad zum Antreiben des Mittelschlauchs umfasst.

Vorteilhafterweise umfasst die Antriebseinheit ein Gehäuse. Dabei kann das Schiebeelement auf einer Linearführung des Gehäuses angeordnet sein. Weiterhin ist denkbar, dass der Katheter in das Gehäuse hineingeführt ist, wobei das Schiebeelement in einem ersten Gehäuseabschnitt mit dem Bewegungsabschnitt des Außenschlauchs zusammenwirkt, und wobei das Schiebeelement in einem zweiten Gehäuseabschnitt mit dem Bewegungsabschnitt des Mittelschlauchs zusammenwirkt. Folglich können der erste Gehäuseabschnitt und der zweite Gehäuseabschnitt nebeneinander quer zur axialen Bewegungsrichtung des Schiebeelements angeordnet sein, wobei das erste Mitnahmemittel mit dem Bewegungsabschnitt des Außenschlauchs im Bereich des ersten Gehäuseabschnitts zusammenwirkt, während das zweite Mitnahmemittel im Bereich des zweiten Gehäuseabschnitts mit dem Bewegungsabschnitt des Mittelschlauchs zusammenwirkt.

Das proximale Ende des Außenschlauchs kann dabei zwischen dem ersten Gehäuseabschnitt und dem zweiten Gehäuseabschnitt liegen. Andererseits ist auch denkbar, dass das proximale Ende des Außenschlauchs im Bereich des ersten Gehäuseabschnitts liegt. Folglich ist denkbar, dass der Katheter in das Gehäuse hineingeführt ist, durch den ersten Gehäuseabschnitt hindurchgeführt ist, und weiter zum zweiten Gehäuseabschnitt geführt ist, wobei das proximale Ende des Außenschlauchs in distaler Richtung vor dem zweiten Gehäuseabschnitt liegt, sodass das zweite Mitnahmemittel des Schiebeelements im Bereich des zweiten Gehäuseabschnitts mit dem Bewegungsabschnitt des Mittelschlauchs zusammenwirken kann.

Vorzugsweise weist der Bewegungsabschnitt des Außenschlauchs und/oder des Mittelschlauchs ein Spiralelement oder ein Zahnstangenelement auf. Dabei ist insbesondere denkbar, dass das Spiralelement radial um den Außenschlauch beziehungsweise den Mittelschlauch verlaufend angeordnet ist. Damit kann eine bessere Bewegungskopplung zwischen Schiebelement und Außenschlauch bzw. Mittelschlauch bereitgestellt werden, indem eine optimale lineare Krafteinleitung ermöglicht wird.

Dabei hat es sich als besonders vorteilhaft herausgestellt, wenn ein Schrumpfschlauch um den Bewegungsabschnitt des Außenschlauchs und/oder um den Bewegungsabschnitt des Mittelschlauchs verlaufend aufgeschrumpft ist, wobei ein Spiralelement zwischen den Schrumpfschlauch und dem Bewegungsabschnitt des Mittelschlauchs bzw. dem Bewegungsabschnitt des Außenschlauchs angeordnet sein kann. Insbesondere, falls ein Spiralelement um den Bewegungsabschnitt verlaufend angeordnet ist, kann dabei eine noch bessere Bewegungskopplung erzielt werden, da das Spiralelement aufgrund des aufgeschrumpften Schrumpfschlauchs in Längsrichtung nicht oder nur wenig elastisch nachgiebig ist.

Gemäß einer besonders bevorzugten Ausführungsform der Erfindung weist das System ein erstes Rückhaltemittel und ein zweites Rückhaltemittel auf, wobei das erste Rückhaltemittel mit dem Bewegungsabschnitt des Außenschlauchs zusammenwirkt und wobei das zweite Rückhaltemittel mit dem Bewegungsabschnitt des Mittelschlauchs zusammenwirkt. Die Rückhaltemittel können dabei im Gehäuse der Antriebseinheit angeordnet sein. Dabei ist diejenige Rückhaltekraft, die das erste Rückhaltemittel auf den Bewegungsabschnitt des Außenschlauchs ausübt in eine Schiebeelementbewegungsrichtung größer als diejenige Mitnahmekraft, die das erste Mitnahmeelement auf den Bewegungsabschnitt des Außenschlauchs ausübt. Weiterhin ist diejenige Rückhaltekraft, die das zweite Rückhaltemittel auf den Bewegungsabschnitt des Mittelschlauchs ausübt in eine Schiebeelementbewegungsrichtung größer als diejenige Mitnahmekraft, die das zweite Mitnahmeelement auf den Bewegungsabschnitt des Mittelschlauchs ausübt. Damit wird bewirkt, dass lediglich eine Bewegungsrichtung des Schiebeelements eine Außenschlauchmitnahmerichtung darstellt, in die eine Bewegungskopplung zwischen dem Bewegungsabschnitt des Außenschlauchs und dem Schiebeelement vorhanden ist sowie das lediglich eine Bewegungsrichtung des Schiebeelements eine Mittelschlauchmitnahmerichtung darstellt, in die eine Bewegungskopplung zwischen dem Bewegungsabschnitt des Mittelschlauchs und dem Schiebeelement vorhanden ist. Somit kann der selbstexpandierbare Stent Stück für Stück kontrolliert freigesetzt werden, indem das Schiebelement hin und her bewegt wird und damit nach und nach der Außenschlauch in proximaler Richtung und der Mittelschlauch in distale Richtung bewegt werden.

Die Außenschlauchmitnahmerichtung kann dabei der Mittelschlauchmitnahmerichtung entsprechen, sodass die Bewegung des Schiebeelements eine synchrone Bewegung des Außenschlauchs in proximaler Richtung und des Mittelschlauchs in distaler Richtung bewirkt. Andererseits ist auch denkbar, dass die Außenschlauchmitnahmerichtung der Mittelschlauchmitnahmerichtung entgegengesetzt ist, sodass der Außenschlauch in proximaler Richtung und der Mittelschlauch in distaler Richtung abwechselnd mittels des Schiebeelements verschiebbar sind. Entgegen der Außenschlauchmitnahmerichtung (Außenschlauchleerlaufrichtung) wird das erste Mitnahmemittel des Schiebeelements, welches mit dem Bewegungsabschnitt des Außenschlauchs zusammenwirkt in einem Leerlauf über den Bewegungsabschnitt bewegt, wobei insbesondere, wenn es sich bei den Mitnahmemitteln um Eingriffzungen handelt, die Eingriffzungen über den Bewegungsabschnitt "gezogen" werden. Dasselbe gilt, wenn das Schiebeelement entgegen der Mittelschlauchmitnahmerichtung (Mittelschlauchleerlaufrichtung) bewegt wird für das zweite Mitnahmemittel. Bei den Rückhaltemitteln kann es sich dabei insbesondere um Fixierzungen handeln. Die Fixierzungen können dabei insbesondere Blechteile sein und sich quer zur axialen Bewegungsrichtung des Schiebelements erstrecken. Dabei wirkt das freie Ende der Fixierzungen mit dem Bewegungsabschnitt des Mittelschlauchs bzw. des Außenschlauchs, insbesondere über die Spiralelemente, zusammen. Entgegen der Mittelschlauch- bzw. Außenschlauchmitnahmerichtung sind die Fixierzungen dabei im Wesentlichen nicht elastisch umbiegbar und blockieren folglich eine Außen- bzw. Mittelschlauchbewegung. Wirken allerdings Querkräfte über den Bewegungsabschnitt des Außen- bzw. Mittelschlauchs, respektive über die daran angeordneten Spiralelemente, in der Außen- bzw. Mittelschlauchmitnahmerichtung an den Fixierzungen, so biegen sich diese elastisch um und geben dabei eine Außen- bzw. Mittelschlauchbewegung frei.

Um eine Sperrwirkung entgegen der Außenschlauchmitnahmerichtung beziehungsweise der Mittelschlauchmitnahmerichtung zu erzielen, ist insbesondere dann, wenn es sich bei den Rückhaltemitteln um Fixierzungen handelt, denkbar, dass ein erstes Anlagemittel für das erste Rückhaltemittel und ein zweites Anlagemittel für das zweite Rückhaltemittel vorgesehen sind, sodass das erste Rückhaltemittel in die Außenschlauchmitnahmerichtung elastisch nachgiebiger ist als entgegen dieser, und so dass das zweite Rückhaltemittel in die Mittelschlauchmitnahmerichtung elastisch nachgiebiger ist als entgegen dieser. Dies wird dadurch erzielt, dass das erste Rückhaltemittel bzw. das zweite Rückhaltemittel am ersten Anlagemittel bzw. am zweiten Anlagemittel anliegen und lediglich nur von den Anlagemitteln elastisch wegbiegbar sind. Insbesondere dann, wenn der erste bzw. der zweite Bewegungsabschnitt ein Spiralelement oder ein Zahnstangenelement aufweist, kann eine besonders effektive Sperrwirkung erzielt werden, da die Rückhaltemittel unmittelbar in die Spiralfedern beziehungsweise die Zahnstangenelemente eingreifen können und dabei eine Blockierung der Bewegungsabschnitte wirksam herbeiführen können.

Dabei hat sich weiterhin als wünschenswert herausgestellt, dass die Außenschlauchmitnahmerichtung und die Mittelschlauchmitnahmerichtung umkehrbar sind. Folglich ist denkbar, dass das erste Anlagemittel eine erste Anlagerippe und eine zweite Anlagerippe für das erste Rückhaltemittel aufweist, und dass das zweite Anlagemittel eine erste Anlagerippe und eine zweite Anlagerippe für das zweite Rückhaltemittel aufweist. In einer ersten Schaltstellung liegen das erste und das zweite Rückhaltemittel jeweils an einer der beiden Anlagerippen an, während das erste Rückhaltemittel und das zweite Rückhaltemittel in der zweiten Schaltstellung jeweils an der anderen der beiden Anlagerippen anliegen. Das erste und das zweite Rückhaltemittel können dabei abhängig oder unabhängig voneinander zwischen der ersten und der zweiten Schaltstellung schaltbar sein. Insbesondere ist denkbar, dass die Rückhaltemittel an einem Rückhaltemittelschiebeelement angeordnet sind. Das Rückhaltemittelschiebeelement kann dabei an einer Linearführung am Gehäuse der Antriebseinheit angeordnet sein, sodass durch eine Bewegung des Rückhaltemittelschiebeelements eine synchrone Schaltung der Rückhaltemittel von der ersten Schaltstellung in die zweite Schaltstellung ermöglicht wird. Zur Freisetzung des Stents wird dabei diejenige Schaltstellung eingenommen, in der der Außenschlauch in proximaler Richtung und der Mittelschlauch in distaler Richtung bewegt werden.

Weiterhin ist denkbar, dass am Schiebeelement ein erstes Anlagemittel zur Anlage des ersten Mitnahmemittels und ein zweites Anlagemittel zur Anlage des zweiten Mitnahmemittels angeordnet sind. Das erste Mitnahmemittel kann folglich am ersten Anlagemittel angelegt werden und ist damit entgegen der Außenschlauchmitnahmerichtung elastisch nachgiebiger als in die Außenschlauchmitnahmerichtung. Dasselbe gilt für das zweite Mitnahmemittel, sodass dieses entgegen der Mittelschlauchmitnahmerichtung elastisch nachgiebiger ist als in diese.

Dabei ist einerseits denkbar, dass Anlagemittel sowohl für die Mitnahmemittel als auch für die Rückhaltemittel vorgesehen sind. Andererseits ist auch denkbar, dass die Rückhaltemittel in beide Bewegungsrichtungen des Schiebeelements eine konstante Rückhaltekraft bereitstellen, während das Vorsehen der Anlagemittel am Schiebeelement bewirkt, dass die Mitnahmekraft des ersten Mitnahmemittels bzw. des zweiten Mitnahmemittels in eine Bewegungsrichtung des Schiebeelements größer ist als in die andere.

Weiterhin ist auch denkbar, dass eine Umkehrung der Außenschlauchmitnahmerichtung beziehungsweise der Mittelschlauchmitnahmerichtung dadurch erreicht wird, dass das erste Anlagemittel beziehungsweise das zweite Anlagemittel des Schiebeelements eine erste und eine zweite Anlagerippe aufweisen, wobei das erste Mitnahmemittel und das zweite Mitnahmemittel zwischen einer ersten und einer zweiten Schaltstellung schaltbar sind, wobei in einer ersten Schaltstellung das erste und das zweite Mitnahmemittel jeweils an einer der beiden Anlagerippen anliegen, und wobei das erste und das zweite Mitnahmemittel in einer zweiten Schaltstellung jeweils an der anderen der beiden Anlagerippen anliegen. Das erste und zweite Mitnahmemittel können dabei abhängig oder unabhängig voneinander zwischen der ersten und der zweiten Schaltstellung schaltbar sein. Dabei ist insbesondere denkbar, dass die Mitnahmemittel an einem verschiebbaren Mitnahmemittelschiebeelement angeordnet sind und abhängig voneinander synchron durch das Mitnahmemittelschiebeelement zwischen der ersten Schaltstellung und der zweiten Schaltstellung verlagerbar sind. Zur Freisetzung des Stents wird dabei diejenige Schaltstellung eingenommen, in der der Außenschlauch in proximaler Richtung und der Mittelschlauch in distaler Richtung bewegt werden.

Gemäß einer weiteren besonders vorteilhaften Ausführungsform der Erfindung, weist das Schiebeelement ein erstes Haltemittel und/oder ein zweites Haltemittel auf, wobei das erste Mitnahmemittel am ersten Haltemittel angeordnet ist und/oder wobei das zweite Mitnahmemittel am zweiten Haltemittel angeordnet ist. Dabei weist das Schiebeelement ein Paar Mitnehmerrippen auf, wobei der erste Haltemittel oder der zweite Haltemittel zwischen den Mitnehmerrippen axial hin und her bewegbar angeordnet ist, sodass die Verschiebungsstrecke des Mittelschlauchs in distaler Richtung und des Außenschlauchs in proximaler Richtung nach einer vollständigen axialen Hin- und Herbewegung des Schiebeelements zwischen einer ersten Endlage und einer zweiten Endlage des Schiebeelements unterschiedlich sind. Es kann folglich eine Übersetzung realisiert werden, was bedeutet, dass unterschiedliche Schiebewege des Außenschlauchs und des Mittelschlauchs realisiert werden können. Damit kann eine besonders präzise Freisetzung des Stents erfolgen. Das Verhältnis des Schiebewegs des Außenschlauchs zum Schiebeweg des Mittelschlauchs, welches die Übersetzung darstellt, kann dabei abhängig von den Stentparametern dadurch geändert werden, dass die axiale Beabstandung der Mitnehmerrippen variiert wird. Dabei ist denkbar, dass die Beabstandung einstellbar ist, oder dass Schiebeelemente mit unterschiedlichen Beabstandungen bereitgestellt werden.

Alternativ ist denkbar, dass das erste Haltemittel oder das zweite Haltemittel ein Paar Mitnehmerrippen aufweist, und dass das Schiebeelement ein Haltemitteleingriffelement aufweist, sodass das Schiebelement zwischen den Mitnehmerrippen axial hin und her bewegbar ist. Auch hier kann eine Übersetzung durch Variieren des Mitnehmerrippenabstands erreicht werden.

Gemäß einer weiteren Ausgestaltung der Erfindung können das erste Rückhaltemittel und das zweite Rückhaltemittel jeweils einen ersten Rückhaltemittelabschnitt und einen zweiten Rückhaltemittelabschnitt aufweisen. Dabei liegen der erste Rückhaltemittelabschnitt und der zweite Rückhaltemittelabschnitt jeweils am ersten Anlagemittel beziehungsweise am zweiten Anlagemittel verlagerbar an. Das erste Rückhaltemittel und das zweite Rückhaltemittel sind dabei zwischen einer ersten Schaltstellung und einer zweiten Schaltstellung schaltbar. In der ersten Schaltstellung ist jeweils einer der beiden Rückhaltemittelabschnitte des Rückhaltemittels beziehungsweise des zweiten Rückhaltemittels mit dem Bewegungsabschnitt des Außenschlauchs beziehungsweise mit dem Bewegungsabschnitt des Mittelschlauchs in Eingriff. In einer zweiten Schaltstellung ist jeweils der andere der beiden Rückhaltemittelabschnitte mit dem Bewegungsabschnitt des Außenschlauchs beziehungsweise dem Bewegungsabschnitt des Mittelschlauchs in Eingriff. Dabei ist denkbar, dass das erste Rückhaltemittel und das zweite Rückhaltemittel an einem Rückhaltemittelstellglied angeordnet sind und folglich synchron zwischen der ersten Schaltstellung und der zweiten Schaltstellung schaltbar sind. Damit kann eine Umkehrung der Außenschlauchmitnahmerichtung und der Mittelschlauchmitnahmerichtung bereitgestellt werden.

Andererseits ist auch denkbar, dass das erste Mitnahmemittel und das zweite Mitnahmemittel jeweils einen ersten Mitnahmemittelabschnitt und einen zweiten Mitnahmemittelabschnitt aufweisen, wobei der erste Mitnahmemittelabschnitt und der Mitnahmemittelabschnitt am ersten Anlagemittel beziehungsweise am zweiten Anlagemittel verlagerbar anliegen. Dabei können die Mitnahmemittel zwischen einer ersten Schaltstellung und einer zweiten Schaltstellung schaltbar sein. Weiterhin ist dabei jeweils einer der beiden Mitnahmemittelabschnitte des ersten Mitnahmemittels bzw. des zweiten Mitnahmemittels mit dem Bewegungsabschnitt des Außenschlauchs bzw. dem Bewegungsabschnitt des Mittelschlauchs in Eingriff. In einer zweiten Schaltstellung ist jeweils der andere der beiden Mitnahmemittelabschnitte mit dem Bewegungsabschnitt des Außenschlauchs bzw. mit dem Bewegungsabschnitt des Mittelschlauchs in Eingriff.

Vorteilhafterweise sind sowohl die Rückhaltemittel als auch die Mitnahmemittel mittels eines Stellglieds derart bewegbar sind, dass durch Betätigen des Stellglieds die Schaltstellung der Rückhaltemittelabschnitte und die Schaltstellung der Mitnahmemittelabschnitte synchron veränderbar sind. Dabei sind die Rückhaltemittelabschnitte jeweils in die Außenschlauchmitnahmerichtung bzw. die Mittelschlauchmitnahmerichtung weg von den Anlagemitteln durchbiegbar, während die Mitnahmemittelabschnitte jeweils entgegen der Außenschlauchmitnahmerichtung bzw. der Mittelschlauchmitnahmerichtung weg von den Anlagemitteln durchbiegbar sind.

Alternativ ist auch denkbar, dass durch eine Bewegung des Stellglieds kein Mitnahmemittelabschnitt bzw. kein Rückhaltemittelabschnitt weggebogen und damit von den Anlagemitteln abgehoben wird. Stattdessen können durch eine Bewegung des Stellglieds Rückhaltemittelbewegungsmittel, sowie Mitnahmemittelbewegungsmittel derart bewegt werden, dass das Rückhaltemittel und das Mitnahmemittel um Drehachsen bzw. gedreht werden, sodass je nach Schaltstellung entweder der erste Rückhaltemittelabschnitt sowie der erste Mitnahmemittelabschnitt oder der zweite Rückhaltemittelabschnitt sowie der zweite Mitnahmemittelabschnitt mit der Spiralfeder bzw. dem Mittelschlauch im Bereich des Bewegungsabschnitts in Eingriff sind. Dabei verlaufen die Drehachsen vorteilhafterweise senkrecht zur axialen Bewegungsrichtung des Schiebelements.

Gemäß einer weiteren Ausgestaltung der Erfindung, ist der Katheter vom zweiten Gehäuseabschnitt zu einem Spülanschluss zu einem gehäuseseitigen Spülanschluss geführt, wobei der Mittelschlauch vor dem Spülanschluss endet, sodass das proximale Ende des Innenschlauchs mit dem Spülanschluss fluiddicht verbindbar ist. Andererseits ist ebenso denkbar, dass proximale Ende des Innenschlauchs den Spülanschluss aufweist. Dabei ist insbesondere denkbar, dass das distale Ende des Innenschlauchs eine Spitze derart aufweist, dass Flüssigkeit, durch den Innenschlauch zum distalen Ende des Innenschlauchs geführt werden kann und zwischen Außenschlauch und Innenschlauch zum proximalen Ende des Außenschlauchs zurückfließen kann. Der Rückflussstrom ist folglich erkennbar am Flüssigkeitsaustritt am proximalen Außenschlauchende. Wenn der Katheter in das Gehäuse hineingeführt ist, ist ebenso denkbar, dass vor dem Eintritt in das Gehäuse eine Öffnung am Außenschlauch derart vorgesehen ist, dass Flüssigkeit durch diese Öffnung austreten kann.

Gemäß einer besonders bevorzugten Ausführungsform der Erfindung wird der Katheter zwischen dem ersten und dem zweiten Gehäuseabschnitt in einem Führungsschlauch geführt. Insbesondere kann der Führungsschlauch dabei bogenförmig vom ersten Gehäuseabschnitt zum zweiten Gehäuseabschnitt verlaufen, damit kann ein Verkanten des Katheters in dem Führungsschlauch beziehungsweise zwischen dem ersten und dem zweiten Gehäuseabschnitt wirkungsvoll verhindert werden. Weiterhin kann der Katheter vom zweiten Gehäuseabschnitt zu einem Spülanschluss in einem bogenförmigen Führungsschlauch geführt sein. Die Führungsschläuche können dabei innerhalb des Gehäuses geführt sein. Alternativ ist denkbar, dass der Katheter bzw. die einzelnen Schläuche in Kanälen im Gehäuse geführt sind, sodass keine Führungsschläuche benötigt werden. Gemäß einer weiteren Ausführungsform der Erfindung kann die Antriebseinheit als Zahnradgetriebe ausgebildet sein. Dabei wirkt ein erstes Zahnrad mit dem Bewegungsabschnitt des Außenschlauchs zusammen und ein zweites Zahnrad wirkt mit dem Bewegungsabschnitt des Mittelschlauchs zusammen. Mittels eines Drehrades kann dabei eine Bewegung des Getriebes erreicht werden, sodass der Mittelschlauch in distaler Richtung und der Außenschlauch in proximaler Richtung bewegt werden. Über ein entsprechend ausgebildetes Getriebe kann auch erreicht werden, dass die Verschiebungsstrecke des Mittelschlauchs in distaler Richtung und des Außenschlauchs in proximaler Richtung nach einer vollständigen Drehung des Drehrads unterschiedlich sind. Dabei ist insbesondere denkbar, dass Zahnstangenelemente oder Spiralfedern an den Bewegungsabschnitten des Mittelschlauchs bzw. des Außenschlauchs angeordnet sind und die Zahnräder in Eingriff mit den Zahnstangenelementen beziehungsweise den Spiralfedern sind.

Weiterhin ist auch denkbar, dass ein erstes und/oder ein zweites Drehrad radial um den ersten bzw. zweiten Bewegungsabschnitt verlaufen, wobei der Bewegungsabschnitt dabei insbesondere von einem Spiralelement umgeben sein kann. Damit kann ein Spindel-Mutter-getriebeartiges Getriebe bereitgestellt werden, wobei das erste und/oder das zweite Drehrad derart mit den Spiralelementen des ersten und/oder des zweiten Bewegungsabschnitts zusammenwirken, dass eine Drehung des ersten oder des zweiten Drehrades in einer Bewegung des Außen- bzw. Mittelschlauchs resultiert. Die eingangs genannte Aufgabe wird auch durch einen Katheter für ein erfindungsgemäßes System gelöst. Ein derartiger Katheter zeichnet sich folglich insbesondere dadurch aus, dass ein Innenschlauch, ein Mittelschlauch und ein Außenschlauch vorgesehen sind, wobei sich der Mittelschlauch durch den Außenschlauch erstreckt, und wobei sich der Innenschlauch durch den Mittelschlauch erstreckt. Dabei kann ein komprimierter Stent in einem distalen Endabschnitt des Katheters radial und um den Innenschlauch verlaufend zwischen dem Innenschlauch und dem Außenschlauch radial komprimiert anordenbar sein, wobei der Mittelschlauch distal am proximalen Ende eines am Katheter angeordneten Stents endet.

Schließlich ist denkbar, dass am distalen Endabschnitt des Mittelschlauchs ein Stenteingreifelement angeordnet ist. Dabei kann sich das Stenteingreifelement ausgehend vom distalen Ende des Mittelschlauchs zwischen dem Innenschlauch und dem komprimierten Stent in distaler Richtung erstrecken, wobei das distale Ende des Stenteingreifelements zwischen dem proximalen Ende und dem distalen Ende des komprimierten Stents liegt. Ferner kann das Stenteingreifelement einen insbesondere distalen Endabschnitt aufweisen, wobei am Endabschnitt ein sich quer zur distalen beziehungsweise proximalen Richtung erstreckendes Eingriffselement vorgesehen sein kann. Das Eingriffselement kann dabei insbesondere mit dem Stent in Eingriff sein. Insbesondere kann das Stenteingreifelement als Hülse oder Röhrchen, insbesondere als Kunststoffröhrchen oder als metallisches Röhrchen, ausgebildet sein. Dabei kann das Eingriffselement als Haken oder als ringbundartige Eingriffsnase ausgebildet sein, welche das Röhrchen in radialer Richtung überragt. Der Haken oder die Nase kann sich dabei um den gesamten Umfang oder um einen Abschnitt des Umfangs erstrecken. Das Röhrchen kann dabei einen verjüngten Bereich aufweisen, an welchem das Eingriffselement angeordnet oder abgeformt ist. Das Eingriffselement kann dabei auch als auf das Röhrchen aufgesetztes Hülsenelement ausgebildet sein.

Dadurch kann eine vorteilhafte Krafteinleitung beim insbesondere distalen Verschieben des Stents erzielt werden, dadurch dass sowohl das distale Ende des Mittelschlauchs als auch das Eingriffselement mit dem Stent in Eingriff sind und eine Bewegung in distaler und/oder proximaler Richtung des Mittelschlauchs auf den Stent übertragen wird und damit der Stent in distaler und/oder proximaler Richtung bewegt und damit auch freisetzt werden kann. Vorzugsweise kann der Stent dann in beide Richtungen durch Umschaltung der Bewegungsrichtung bewegt werden. Eine Bewegung kann so erfolgen, dass der Stent vollständig freigesetzt wird. Zusätzlich kann der Innenschlauch stabilisiert werden und gegen ein Abknicken geschützt werden, so dass der Innenschlauch durch den Stent hindurch aus dem Blutgefäß abgezogen werden kann.

Weiterhin wird die eingangs genannte Aufgabe auch durch eine Antriebseinheit für ein erfindungsgemäßes System gelöst. Eine derartige Antriebseinheit zeichnet sich folglich insbesondere dadurch aus, dass ein Schiebeelement an einer Linearführung des Gehäuses der Antriebseinheit axial hin und her bewegbar angeordnet sein kann.

Schließlich wird die eingangs genannte Aufgabe auch durch ein Verfahren zum Einführen eines komprimierten, selbstexpandierbaren Stents in insbesondere ein Blutgefäß und zur Freisetzung des Stents in dem insbesondere einen Blutgefäß unter Verwendung eines erfindungsgemäßen Systems gelöst. Dabei wird der Stent zunächst mittels des Katheters an der Bestimmungsposition zugeführt. In einem zweiten Schritt wird der Stent dann freigesetzt, indem der Außenschlauch in proximaler Richtung und der Mittelschlauch in distaler Richtung abwechselnd oder synchron verschoben werden. Dabei wird der Stent nach und nach freigesetzt, wobei das distale Ende des Stents während der Freisetzung seine Bestimmungsposition beibehält.

Weitere Einzelheiten und vorteilhafte Ausgestaltungen der Erfindung sind der nachfolgenden Beschreibung zu entnehmen, anhand derer die Erfindung näher beschrieben und erläutert wird. Es zeigen:
- Figur 1: schematische Darstellung des Freisetzungsvorgangs eines selbstexpandierenden Stents am distalen Ende eines Katheters;
- Figur 2: Schrägansicht eines Systems mit einem Katheter und einer Antriebseinheit;
- Figur 3: Explosionsdarstellung des Systems gemäß Fig. 2;
- Figur 4a: Draufsicht auf das System;

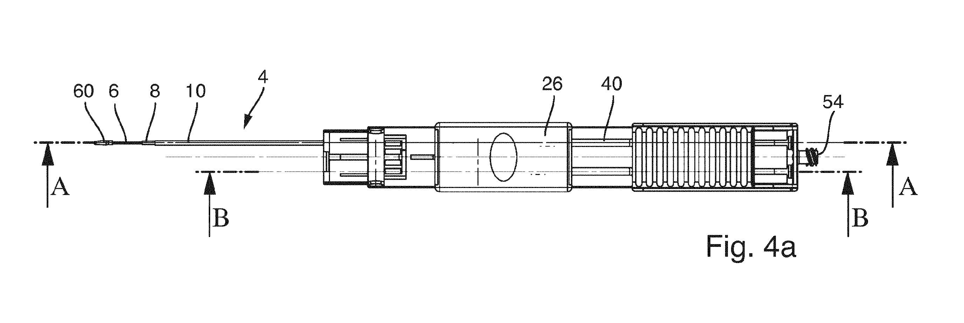

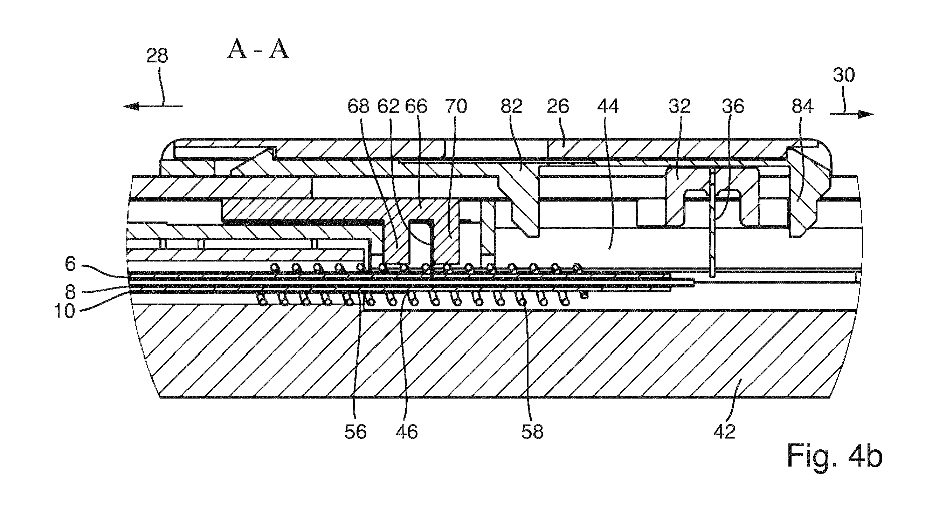

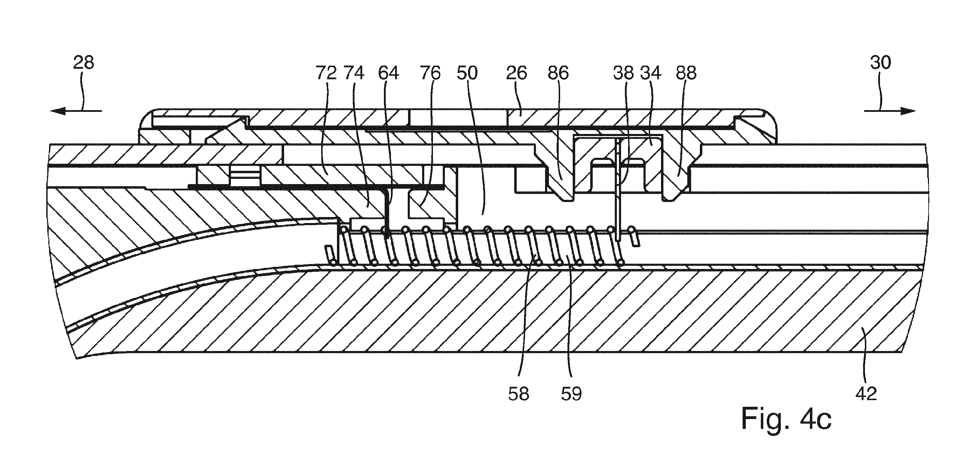

- Figur 4: Schematischer Ausschnitt eines Querschnitts entlang der Linie A-A des Systems gemäß Fig. 4a;
- Figur 4c: Schematischer Ausschnitt eines Querschnitts entlang der Linie B-B des Systems gemäß Fig. 4a;

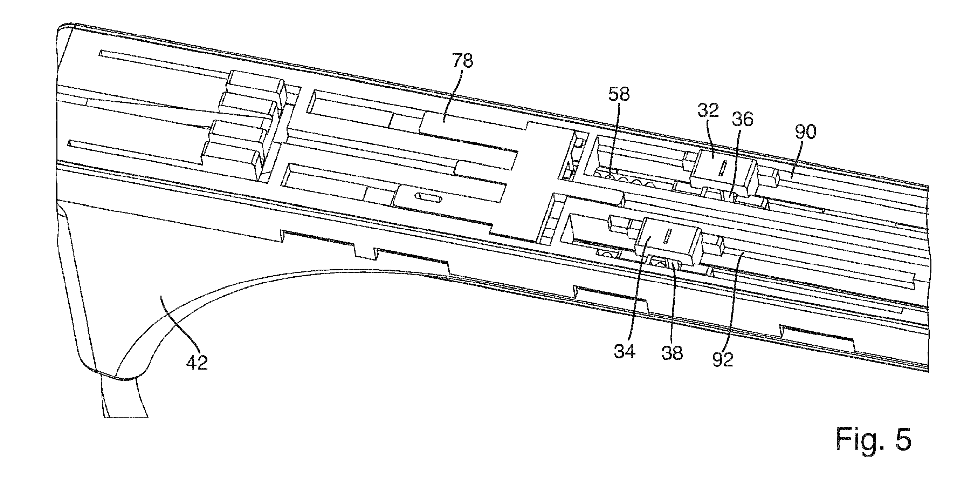

- Figur 5: schematische Schrägansicht eines Teils des Systems;

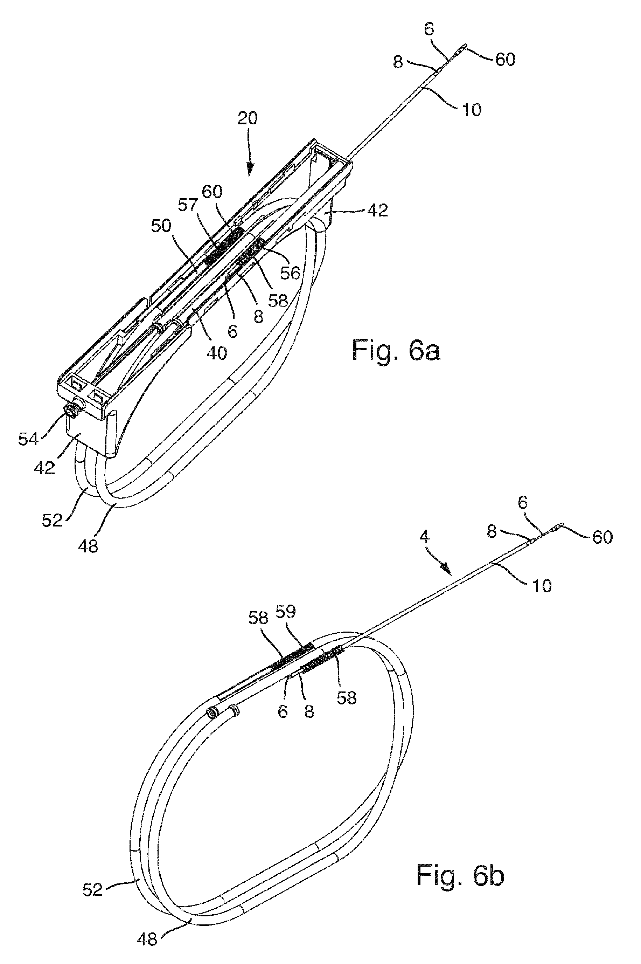

- Figur 6a: schematische Schrägansicht eines weiteren Teils des Systems;
- Figur 6b: schematische Schrägansicht gemäß Fig. 6a, ohne Gehäuse;

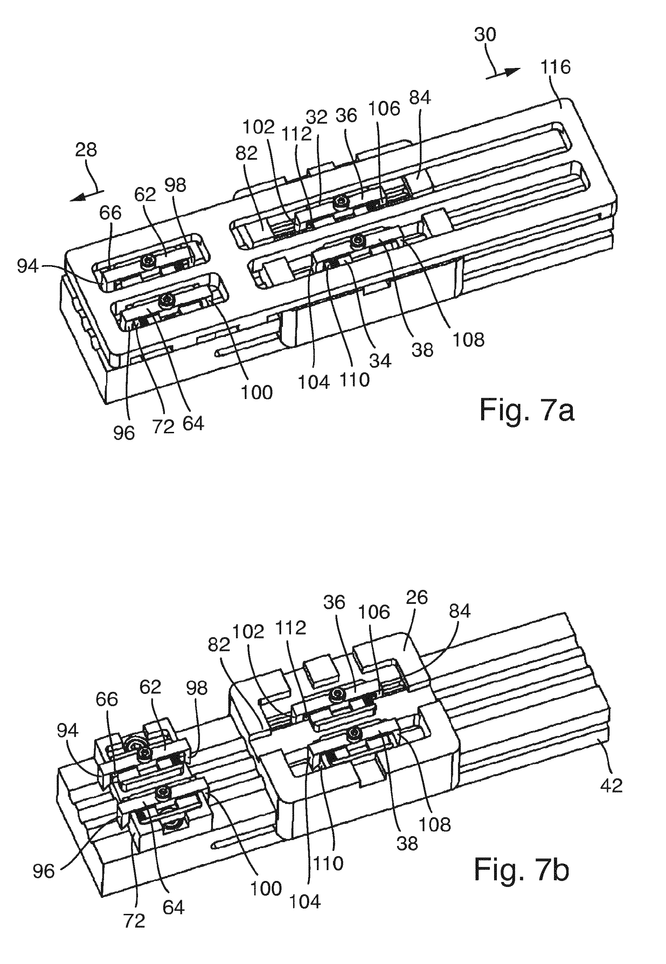

- Figur 7a: schematische Schrägansicht eines Ausschnitts einer weiteren Ausführungsform der Antriebseinheit;
- Figur 7b: schematische Schrägansicht wie in Figur 7a, allerdings ohne Stellglied;

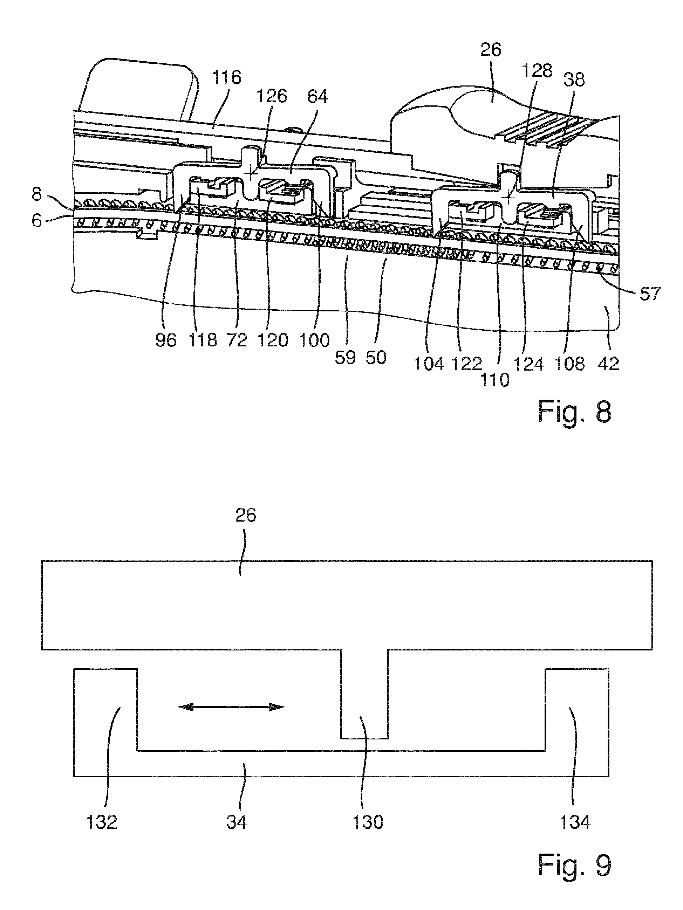

- Figur 8: schematische Seitenansicht des zweiten Gehäuseabschnitts einer weiteren Ausführungsform der Antriebseinheit;
- Figur 9: schematische Seitenansicht einer weiteren Ausführungsform der Antriebseinheit;

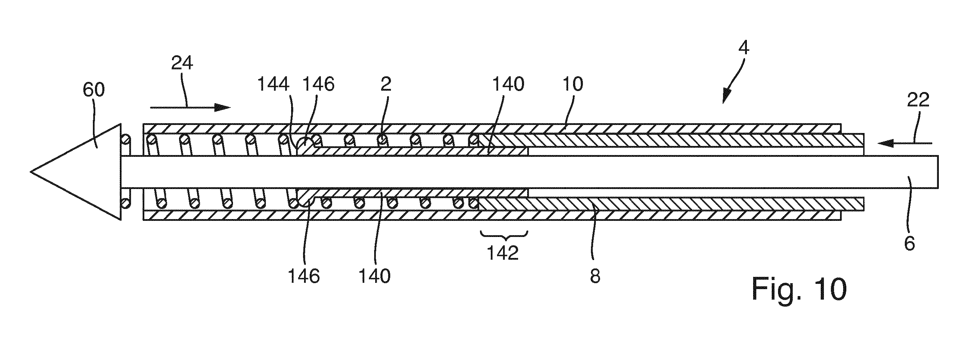

- Figur 10: schematische geschnittene Seitenansicht des distalen Endbereichs eines Katheters gemäß einer weiteren Ausführungsform;

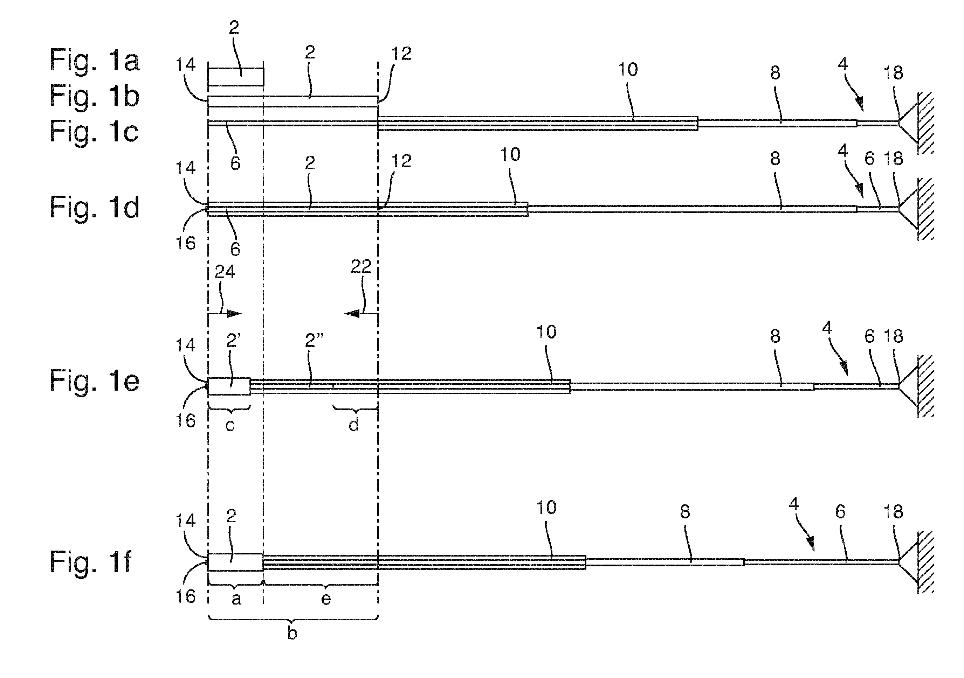

Die Figur 1 zeigt einen selbstexpandierten, geflochtenen Stent 2 (Fig. 1a) und eben diesen Stent 2 (Fig. 1b) in seiner komprimierten, selbstexpandierbaren Form. Folglich ist der Stent in seiner komprimierten Form radial verengt und im Vergleich zur selbstexpandierten Form deutlich länger. Wie der Figur 1 zu entnehmen ist, ist die Länge a des selbstexpandierten Stents 2 deutlich kleiner als die Länge b des komprimierten Stents 2. Die Figur 1c zeigt einen Katheter 4 mit einem Innenschlauch 6, einem Mittelschlauch 8 und einem Außenschlauch 10, wobei sich der Mittelschlauch 8 durch den Außenschlauch 10 erstreckt und sich der Innenschlauch 6 durch den Mittelschlauch 8 erstreckt. In Figur 1d ist zu sehen, dass der komprimierte Stent 2 in einem distalen Endabschnitt des Katheters 4 radial um den Innenschlauch 6 verlaufend zwischen dem Innenschlauch 6 und dem Außenschlauch 10 radial komprimiert angeordnet ist und der Mittelschlauch 8 distal am proximalen Ende 12 des komprimierten Stents 2 endet. Dabei befindet sich das distale Ende 14 des komprimierten Stents 2 in seiner distalen Bestimmungsposition 16. Bei dieser distalen Bestimmungsposition 16 handelt es sich insbesondere um diejenige Position in einem Blutgefäß, an der der selbstexpandierbare Stent 2 freigesetzt werden soll. Wie weiterhin zu erkennen ist, ist der Innenschlauch 6 mit seinem proximalen Ende 18 fixiert gehalten.

In der Figur 1e ist der Freisetzungsvorgang des selbstexpandierbaren Stents gezeigt. Dabei ist der Außenschlauch in proximaler Richtung um eine Distanz c relativ zum Innenschlauch 6 und zum distalen Ende des Katheters 4 beziehungsweise des Stents 2 in proximaler Richtung verschoben, während der Mittelschlauch 8 um eine Distanz d relativ zum Innenschlauch 6 und zum distalen Ende des Katheters 4 beziehungsweise des Stents 2. Dabei ist ein erster Abschnitt 2' des Stents 2 bereits freigesetzt, während ein zweiter Abschnitt 2'' des Stents 2 komprimiert zwischen dem Außenschlauch 10 und dem Innenschlauch 6 des Katheters 4 angeordnet ist. Die Distanz d ist dabei größer als die Distanz c. In Figur 1f ist der freigesetzte Stent 2 zu erkennen. Dabei ist der Außenschlauch 10 um die Distanz a, welche der Länge des selbstexpandierten Stents 2 entspricht in proximaler Richtung verschoben, während der Mittelschlauch 8 um die Distanz e in distaler Richtung verschoben ist. Dabei kann die Länge a beispielsweise 200 mm betragen, während die Länge e 400 mm betragen kann, sodass der komprimierte Stent im Katheter eine Länge von 600 mm einnimmt. Wie in der Figur 1 deutlich zu erkennen ist, ist nach der Freisetzung die Bestimmungsposition 16 des distalen Endes 16 des Stents 2 beibehalten.

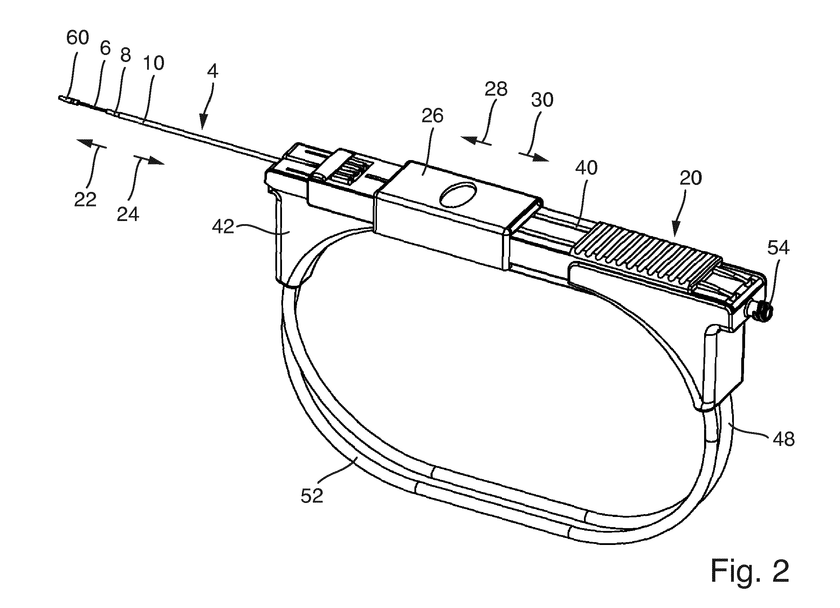

Wie in Figur 2 dargestellt, ist im proximalen Bereich des Katheters eine Antriebseinheit 20 vorgesehen, mittels welcher der Außenschlauch 10 in proximaler Richtung 24 und der Mittelschlauch 8 in distaler Richtung 22 synchron oder zeitlich nacheinander verschiebbar sind. Die Antriebseinheit 20 umfasst dabei ein Schiebeelement 26, welches in den Figuren 2 bis 4a besonders deutlich zu erkennen ist. Dabei ist das Schiebeelement 26 in eine erste Schiebeelementbewegungsrichtung 28 und eine zweite Schiebeelementbewegungsrichtung 30 axial hin und her bewegbar.

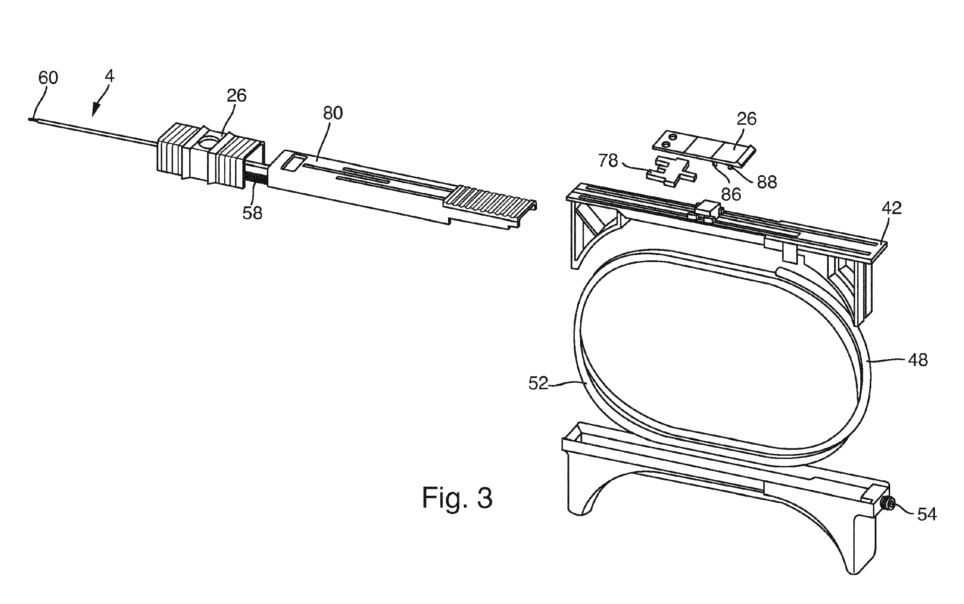

Wie den Figuren 3 bis 5 zu entnehmen ist, weist das Schiebeelement 26 ein erstes Haltemittel 32 und ein zweites Haltemittel 34 auf, wobei am ersten Haltemittel 32 das erste Mitnahmemittel 36 und am zweiten Haltemittel 34 das zweite Mitnahmemittel 38 angeordnet sind. Die Mitnahmemittel 36, 38 sind als Eingriffzungen ausgebildet. Das Schiebeelement 26 ist dabei, wie insbesondere den Figuren 2 und 4a zu entnehmen ist, in einer Linearführung 40 angeordnet, wobei das Gehäuse 42 der Antriebseinheit 20 die Linearführung 40 aufweist.

Wie den Figuren 2 und 4a weiter zu entnehmen ist, wird der Katheter 4 in das Gehäuse 42 der Antriebseinheit 20 hineingeführt. Im Gehäuse 42 wird der Katheter 4 dann zuerst einem ersten Gehäuseabschnitt 44 zugeführt, welcher in Figur 4b gezeigt ist. Dabei liegt das proximale Ende 46 des Außenschlauchs 10 im Bereich des ersten Gehäuseabschnitts 44.

Wie den Figuren 2 und 6 (Schläuche in den Figuren teilweise nicht oder nur abschnittsweise gezeigt) zu entnehmen ist, wird der Katheter 4 ausgehend vom ersten Gehäuseabschnitt 44 durch einen Führungsschlauch 48 bogenförmig zum zweiten Gehäuseabschnitt 50 geführt. Ausgehend vom zweiten Gehäuseabschnitt 50 wird der Innenschlauch 6 durch einen zweiten bogenförmigen Führungsschlauch 52 zu einem Spülanschluss 54 geführt, wobei das proximale Ende des Innenschlauchs 6 mit dem Spülanschluss 54 fluiddicht verbunden ist.

Über den Spülanschluss 54 kann Flüssigkeit dem distalen Endbereich des Katheters 4 zugeführt werden. Dabei weist der Innenschlauch 6 eine Spitze 60 auf, sodass der Außenschlauch 10 mit dem Innenschlauch 6 derart fluidisch verbindbar ist, dass Fluid durch den Innenschlauch 6 dem distalen Ende des Katheters 4 zuführbar ist und zwischen dem Außenschlauch 10 und dem Mittelschlauch 8 und/oder dem Mittelschlauch 8 und dem Innenschlauch 6 aus dem Katheter 4 abführbar ist.

Der Außenschlauch 10 weist im Bereich des ersten Gehäuseabschnitts 44 einen Bewegungsabschnitt 56 auf, wobei ein Spiralelement 58 radial um den Bewegungsabschnitt 56 des Außenschlauchs 10 verlaufend angeordnet ist. Dabei ragt das Spiralelement 58 über das proximale Ende 46 des Außenschlauchs 10 in proximaler Richtung hinaus.

Wie in der Figur 4c lediglich andeutungsweise gezeigt ist, weist der Mittelschlauch 8 (Schläuche in der Figur nicht gezeigt) im Bereich des zweiten Gehäuseabschnitts 50 einen Bewegungsabschnitt 59 auf, wobei um diesen Bewegungsabschnitt 59 wiederum ein Spiralelement 57 radial verlaufend angeordnet ist.

Der Mittelschlauch 8 kann dabei ähnlich wie der Außenschlauch 10 im Bereich des zweiten Gehäuseabschnitts 50 enden. Andererseits ist denkbar, dass der Mittelschlauch 8 erst in einem Bereich zwischen dem zweiten Gehäuseabschnitt 50 und dem Spülanschluss 54 des Innenschlauchs 6 endet.
Wie in den Figuren 4b und 4c besonders deutlich zu erkennen ist, wirken bei einer axialen Hin- und Herbewegung des Schiebeelements 26 die Mitnahmemittel 36, 38 mit den Spiralelementen 57, 58 zusammen und wirken damit aufgrund der Anordnung der Spiralelemente 57, 58 am Außenschlauch 10 bzw. am Mittelschlauch 8 mit dem Außenschlauch 10 und dem Mittelschlauch 8 zusammen.

Dabei weist das Gehäuse 42 der Antriebseinheit 20 ein erstes Rückhaltemittel 62 in Form einer Fixierzunge und ein zweites Rückhaltemittel 64 in Form einer Fixierzunge auf. Dabei wirkt das Rückhaltemittel 62 über die Spiralfeder 58 mit dem Außenschlauch 10 zusammen, während das zweite Rückhaltemittel 64 über die Spiralfeder 57 mit dem Mittelschlauch 8 zusammenwirkt. Weiterhin weist das Gehäuse 42 im Bereich des ersten Gehäuseabschnitts 44 ein erstes Anlagemittel 66 mit einer ersten Anlagerippe 68 und einer zweiten Anlagerippe 70 auf. Im Bereich des zweiten Gehäuseabschnitts 50 weist das Gehäuse 42 ein zweites Anlagemittel 72 mit einer ersten Anlagerippe 74 und einer zweiten Anlagerippe 78 auf. Die ersten Anlagenrippen 68, 74 sind dabei in distaler Richtung bezüglich der zweiten Anlagerippen 70, 78 des ersten Gehäuseabschnitts 44 und des zweiten Gehäuseabschnitts 50 angeordnet.

Weiterhin sind die Rückhaltemittel 62, 64 an einem Rückhaltemittelschiebeelement 78, welches in der Figur 5 besonders deutlich zu erkennen ist, angeordnet. Dabei ist das Rückhaltemittelschiebeelement 78 über einen Richtungseinsteller 80, welcher in der Figur 3 gezeigt ist, zwischen einer ersten Schaltstellung und einer zweiten Schaltstellung verlagerbar. In den Figuren 4b und 4c ist eine der beiden Schaltstellungen gezeigt. Im ersten Gehäuseabschnitt 44 liegt das erste Rückhaltemittel 62 an der zweiten Anlagerippe 70 an. Das erste Rückhaltemittel 62 ist folglich in die erste Schiebeelementbewegungsrichtung 28 elastisch nachgiebig, in dem es von der zweiten Anlagerippe 70 wegbiegbar ist, während in die zweite Schiebeelementbewegungsrichtung 30 auf Grund der Anlage an der zweiten Anlagerippe 70 keine beziehungsweise eine lediglich geringe elastische Nachgiebigkeit des Rückhaltemittels 62 vorhanden ist. Dies bewirkt, dass die Rückhaltekraft, die das erste Rückhaltemittel 62 auf den Bewegungsabschnitt 56 des Außenschlauchs 10 über das Spiralelement 58 ausübt in die zweite Schiebeelementbewegungsrichtung 30 größer ist als diejenige Mitnahmekraft, die das erste Mitnahmeelement 36 auf den Bewegungsabschnitt 56 des Außenschlauchs 10 über die Spiralfeder 58 ausübt. In die erste Schiebeelementbewegungsrichtung 28 ist die Rückhaltekraft, die das erste Rückhaltemittel 62 ausübt aufgrund der elastischen Nachgiebigkeit kleiner als die Mitnahmekraft, die das erste Mitnahmeelement 36 auf den Bewegungsabschnitt 56 des Außenschlauchs 10 über die Spiralfeder 58 ausübt. Damit ist lediglich in eine Schiebeelementbewegungsrichtung des Schiebeelements 26 eine Bewegungskopplung zwischen den Bewegungsabschnitt 56 des Außenschlauchs 10 und dem Schiebeelement 26 vorhanden. Im zweiten Gehäuseabschnitt liegt das zweite Rückhaltemittel 64 an der ersten Anlagerippe 74 an, sodass dass eine Bewegungskopplung in die zweite Schiebeelementbewegungsrichtung 30 vorhanden ist.

Wird über den Richtungseinsteller 80 das Rückhalteschiebeelement 78 verlagert, sodass sich das erste Rückhalteelement 62 und das zweite Rückhalteelement 64 in der zweiten Schaltstellung befinden, an der das erste Rückhalteelement 62 an der ersten Anlagerippe 68 und das zweite Rückhalteelement 64 an der zweiten Anlagerippe 76 anliegen, so wird die Richtung der Bewegungskopplung umgekehrt. Damit ist in der zweiten Schaltstellung eine Bewegungskopplung zwischen dem ersten Mitnahmeelement 36 und dem Außenschlauch 10 in die zweite Schiebeelementbewegungsrichtung 30 vorhanden, während zwischen dem zweiten Mitnahmeelement 38 und dem Mittelschlauch 8 eine Bewegungskopplung in die erste Schiebeelementbewegungsrichtung 28 vorhanden ist.

Wie den Figuren 4b und 4c weiter zu entnehmen ist, weist das Schiebeelement 26 im Bereich des ersten Gehäuseabschnitts 44 eine erste Mitnahmerippe 82 und eine zweite Mitnahmerippe 84 auf. Weiterhin weist das Schiebeelement 26 im Bereich des zweiten Gehäuseabschnitts 50 eine erste Mitnahmerippe 86 und eine zweite Mitnahmerippe 88 auf. Wie insbesondere der Figur 5 zu entnehmen ist, sind das erste Haltemittel 32 und das zweite Haltemittel 34 jeweils auf einer gehäuseseitigen Linearführung 90, 92 axial hin- und her bewegbar angeordnet. Während das zweite Haltemittel 34 von den Mitnehmerrippen 86, 88 spielfrei gehalten wird, ist zwischen dem ersten Haltemittel 32 und den Mitnehmerrippen 82, 84 ein Axialspiel vorhanden. Das bedeutet, dass die Verschiebungsstrecke des Mittelschlauchs 8 in der ersten Schaltstellung in distaler Richtung und des Außenschlauchs 10 in proximaler Richtung nach einer vollständigen Hin- und Herbewegung des Schiebeelements 26 zwischen einer ersten Endlage und einer zweiten Endlage unterschiedlich sind. Dabei ist insbesondere denkbar, dass die Strecke, die der Mittelschlauch 8 in distaler Richtung bewegt wird doppelt so groß ist als die Strecke, die der Außenschlauch 10 in proximaler Richtung nach einer vollständigen Hin- und Herbewegung des Schiebeelements zurückgelegt hat.

Alternativ kann, wie in Fig. 9 gezeigt ist, das erste und/oder zweite Haltemittel eine erste Mitnehmerrippe 132 und eine zweite Mitnehmerrippe 134 aufweisen. Dabei weist das Schiebelement 26 ein Haltemitteleingriffelement 130 auf. Auch damit kann eine Übersetzung bereitgestellt werden, indem das Haltemitteleingriffelement 130 und damit auch das Schiebelement 26 axial zwischen den Mitnehmerrippen 132, 134 bewegbar ist. Das Haltemittel 34 wird dabei erst folglich erst dann bewegt, wenn das Haltemitteleingriffelement 130 die erste Mitnehmerrippe 132 bzw. zweite Mitnehmerrippe 134 erreicht hat.

Zur Freisetzung des Stents 2, gemäß der Ausführungsform, die in den Figuren 1 bis 6b dargestellt ist, muss zunächst die Schaltstellung der Rückhaltemittel 62, 64 verändert werden. Das bedeutet, dass zur Freisetzung zunächst das Rückhaltemittelschiebelement 78 mittels des Richtungseinstellers 80 in die andere Schaltstellung bewegt werden muss. Danach liegt das Rückhaltemittel 62 an der ersten Anlagerippe 68 an, während das Rückhaltemittel 64 an der zweiten Anlagerippe 76 anliegt. Zur Freisetzung wird dann das Schiebelement 26 axial zwischen der ersten Schiebelementbewegungsrichtung 28 und der zweiten Schiebelementbewegungsrichtung 30 hin und her bewegt. Dabei wird der Außenschlauch 10 in proximaler Richtung bewegt, während der Mittelschlauch 8 in distale Richtung bewegt wird. Somit wird der Stent 2 nach und nach, wie in Figur 1f gezeigt, freigesetzt. Die Bewegung des Außenschlauch 10 und des Mittelschlauchs 8 findet dabei zeitlich nacheinander, abwechselnd statt.

Die Figuren 7a und 7b zeigen eine weitere Ausführungsform der Antriebseinheit 20. Dabei weisen das erste Rückhaltemittel 62 und das zweite Rückhaltemittel 64 jeweils einen ersten Rückhaltemittelabschnitt 94, 96 und einen zweiten Rückhaltemittelabschnitt 98, 100. Weiterhin weisen das erste Mitnahmemittel 36 und das zweite Mitnahmemittel 38 jeweils einen ersten Mitnahmemittelabschnitt 102, 104 und einen zweiten Mitnahmemittelabschnitt 106, 108 auf. Die ersten Rückhaltemittelabschnitte 94, 96 liegen dabei in distaler Richtung vor den zweiten Rückhaltemittelabschnitten 98, 100. Dabei liegen die ersten Rückhaltemittelabschnitte 94, 96 derart am ersten Anlagemittel 66 beziehungsweise am zweiten Anlagemittel 72 an, dass diese in Richtung der ersten Schiebeelementbewegungsrichtung 28 von dem Anlagemittel 72 elastisch nachgiebig wegbewegbar sind. Umgekehrt liegen die zweiten Rückhaltemittelabschnitte 98, 100 am ersten Anlagemittel 66 beziehungsweise am zweiten Anlagemittel 72 derart an, dass diese in die zweite Schiebeelementbewegungsrichtung 30 elastisch nachgiebig wegbiegbar sind. Das erste Mitnahmemittel 36 und das zweite Mitnahmemittel 38 sowie die dazugehörigen Anlagemittel 110, 112 weisen einen korrespondierenden Aufbau auf. Dabei ist in einer ersten Schaltstellung der erste Rückhaltemittelabschnitt 94 des ersten Rückhaltemittels 62 mit dem ersten Bewegungsabschnitt 56 des Außenschlauchs 10 über die Spiralfeder 58 in Eingriff, während der zweite Rückhaltemittelabschnitt 100 des zweiten Rückhaltemittels 64 mit dem Bewegungsabschnitt 59 des Mittelschlauchs 8 über die Spiralfeder 57 in Eingriff ist. Weiterhin ist der erste Mitnehmerabschnitt 102 des ersten Mitnahmemittels 36 mit dem Bewegungsabschnitt 56 des Außenschlauchs 10 in Eingriff, während der zweite Mitnahmeabschnitt 108 des zweiten Mitnahmemittels 38 mit dem Bewegungsabschnitt 59 des Mittelschlauchs 8 über die Spiralfeder 57 in Eingriff ist.

Dabei ist der erste Halteabschnitt 32 als Anlagemittel 112 auf einer Linearführung 114 axial mit einem Axialspiel zwischen zwei Mitnehmerrippen 82, 84 bewegbar ausgebildet, während der zweite Halteabschnitt 34 als Anlagemittel 110 ausgebildet ist und fest mit dem Schiebeelement 26 verbunden ist. Weiterhin sind die Rückhaltemittel 62, 64 und die Mitnahmemittel 36, 38 mittels eines Stellglieds 116 zwischen der ersten und der zweiten Schaltstellung verlagerbar. Weiterhin kann mittels des Stellglieds 116 eine dritte Schaltstellung eingenommen werden, die Neutralstellung, in der sowohl die ersten Rückhaltemittelabschnitte 94, 96 als auch die zweiten Rückhaltemittelabschnitte 98, 100, sowie die ersten Mitnahmemittelabschnitte 102, 104 und die zweiten Mitnahmemittelabschnitte 106, 108 mit dem Bewegungsabschnitt 56 des Außenschlauchs 10 beziehungsweise dem Bewegungsabschnitt 59 des Mittelschlauchs 8 über die Spiralfedern 57, 58 in Eingriff sind, sodass in keine Schiebeelementbewegungsrichtung 28, 30 eine Bewegungskopplung zwischen dem Schiebeelement 26 und dem Außenschlauch 10 sowie dem Mittelschlauch 8 vorhanden ist.

Um den Stent 2, gemäß der Ausführungsform, die in den Figuren 7a und 7b dargestellt ist, freizusetzen, wird zunächst mittels des Stellglieds 116 die passende Schaltstellung eingenommen. In dieser Schaltstellung sind der zweite Rückhaltemittelabschnitt 98 des ersten Rückhaltemittels 62 und der zweite Mitnahmemittelabschnitt 106 des ersten Mitnahmemittels 32 mit dem Bewegungsabschnitt des Außenschlauchs 10 in Eingriff. Weiterhin sind in dieser Schaltstellung der erste Rückhaltemittelabschnitt 96 des zweiten Rückhaltemittels 64 und der erste Mitnahmemittelabschnitt 104 des zweiten Mitnahmemittels 38 mit dem Bewegungsabschnitt des Mittelschlauchs 8 in Eingriff. Zur Freisetzung wird dann das Schiebelement 26 axial zwischen der ersten Schiebelementbewegungsrichtung 28 und der zweiten Schiebelementbewegungsrichtung 30 hin und her bewegt. Dabei wird der Außenschlauch 10 in proximaler Richtung bewegt, während der Mittelschlauch 8 in distale Richtung bewegt wird. Die Bewegung des Außenschlauch 10 und des Mittelschlauchs 8 findet dabei zeitlich nacheinander, abwechselnd statt.

Im Gegensatz zu der in den Figuren 7a und 7b gezeigten Ausführungsform, wird gemäß der Figur 8 durch eine Bewegung des Stellglieds 116 kein Mitnahmemittelabschnitt 102, 104, 106, 108 bzw. kein Rückhaltemittelabschnitt 94, 96, 98, 100 weggebogen und damit von den Anlagemitteln 66, 72, 110, 112 abgehoben. Stattdessen werden durch eine Bewegung des Stellglieds 116 Rückhaltemittelbewegungsmittel 118, 120 sowie Mitnahmemittelbewegungsmittel 122, 124 derart bewegt, dass das Rückhaltemittel 64 und das Mitnahmemittel 38 um die Drehachsen 126 bzw. 128 gedreht werden, sodass je nach Schaltstellung entweder der erste Rückhaltemittelabschnitt 96 sowie der erste Mitnahmemittelabschnitt 104 oder der zweite Rückhaltemittelabschnitt 100 sowie der zweite Mitnahmemittelabschnitt 108 mit der Spiralfeder 57 bzw. dem Mittelschlauch 8 im Bereich des Bewegungsabschnitts 59 in Eingriff sind. Dabei verlaufen die Drehachsen 126 bzw. 128 senkrecht zur axialen Bewegungsrichtung des Schiebelements 26.

Figur 10 zeigt eine weitere Variante des Katheters 4. Dabei ist am distalen Endabschnitt 142 des Mittelschlauchs ein Stenteingreifelement 140 angeordnet. Ausgehend vom distalen Ende des Mittelschlauchs 8 erstreckt sich das Stenteingreifelement 140 dabei in distaler Richtung 24 zwischen dem Innenschlauch 6 und dem komprimierten Stent 2. Das distale Ende 144 des Stenteingreifelements liegt zwischen dem proximalen Ende 12 und dem distalen Ende 14 des komprimierten Stents 2. Am distalen Endabschnitt des Stenteingreifelements 140 im Bereich des freien Endes 144 erstreckt sich quer zur distalen Richtung 22 beziehungsweise zur proximalen Richtung 24 ein Eingriffselement 146. Das Stenteingreifelement 140 ist als Stahlröhrchen ausgebildet, während das Eingriffselement 146 als Eingriffsnase ausgebildet, welches sich über den gesamten Außenumfang des Stahlröhrchens erstreckt und den Außenumfang in radialer Richtung überragt. Dabei ist das Eingriffselement mit dem Stent 2 in Eingriff.

Das Stenteingreifelement 140 trägt folglich zur Krafteinleitung bei, wenn der Mittelschlauch 8 in distaler Richtung 22 verschoben wird. Dadurch kann die Radialkraft beim Zusammendrücken des Stents durch die Außenschlauchbewegung reduziert werden. Ferner wird der Innenschlauch 6 gestützt, so dass nach dem Freisetzen des Stents 2 ein Knicken des Innenschlauchs 6 wirksam verhindert werden kann.

Dabei ist auch denkbar, dass das Eingriffselement 146 mit dem Stent 2 derart in Eingriff steht, dass der Stent 2 bei verschieben des Eingriffselements 140 in distaler als auch in proximaler Richtung bewegt werden kann.

## Patentansprüche

1. System zum Einführen eines komprimierten, selbstexpandierbaren Stents (2) in insbesondere ein Blutgefäß und zur Freisetzung des Stents (2) in dem insbesondere einen Blutgefäß, umfassend einen Katheter (4) mit einem Innenschlauch (6), einem Mittelschlauch (8) und einem Außenschlauch (10), wobei sich der Mittelschlauch (8) durch den Außenschlauch (10) erstreckt und sich der Innenschlauch (6) durch den Mittelschlauch (8) erstreckt, wobei der komprimierte Stent (2) in einem distalen Endabschnitt des Katheters (4) radial um den Innenschlauch (6) verlaufend zwischen dem Innenschlauch (6) und dem Außenschlauch (10) radial komprimiert angeordnet ist und der Mittelschlauch (8) distal am proximalen Ende (14) des Stents (2) endet, und wobei zur Freisetzung des Stents (2) der Außenschlauch (10) in proximaler Richtung (24) und der Mittelschlauch (8) in distaler Richtung (22) derart verschiebbar sind, dass das distale Ende (14) des Stents (2) während der Freisetzung seine Bestimmungsposition (16) beibehält.

2. System nach Anspruch 1, **dadurch gekennzeichnet, dass** im proximalen Bereich des Katheter (4) eine Antriebseinheit (20) vorgesehen ist, mittels welcher der Außenschlauch (10) in proximaler Richtung (24) und der Mittelschlauch (8) in distaler Richtung (22) synchron oder abwechselnd verschiebbar sind, wobei die Antriebseinheit (20) mit einem Bewegungsabschnitt (56) des Außenschlauchs (10) und mit einem Bewegungsabschnitt (60) des Mittelschlauchs (8) zusammenwirkt.

3. System nach Anspruch 2, **dadurch gekennzeichnet, dass** die Antriebseinheit (20) ein Schiebeelement (26) umfasst, wobei das Schiebeelement (26) in eine erste Schiebeelementbewegungsrichtung (28) und eine zweite Schiebeelementbewegungsrichtung (30) axial hin und her bewegbar ist, und wobei am Schiebelement (26) ein erstes Mitnahmemittel (36) und ein zweites Mitnahmemittel (38) angeordnet sind, wobei das erste Mitnahmemittel (36) mit dem Bewegungsabschnitt (56) des Außenschlauchs (10) zusammenwirkt, und wobei das zweite Mitnahmemittel (38) mit dem Bewegungsabschnitt (59) des Mittelschlauchs (8) zusammenwirkt.

4. System nach Anspruch 3, **dadurch gekennzeichnet, dass** die Antriebseinheit (20) ein Gehäuse (42) umfasst, wobei der Katheter (4) in das Gehäuse (42) hineingeführt ist, wobei das Schiebelement in einem ersten Gehäuseabschnitt (44) mit dem Bewegungsabschnitt (56) des Außenschlauchs (10) zusammenwirkt, und wobei das Schiebeelement (26) in einem zweiten Gehäuseabschnitt (50) mit dem Bewegungsabschnitt (59) des Mittelschlauchs (8) zusammenwirkt.

5. System nach einem der Ansprüche 2 bis 4, **dadurch gekennzeichnet, dass** ein Spiralelement (57, 58) oder ein Zahnstangenelement um den Bewegungsabschnitt (56) des Außenschlauchs (10) und/oder um den Bewegungsabschnitt (59) des Mittelschlauchs (8) verlaufend angeordnet ist.

6. System nach Anspruch 5, **dadurch gekennzeichnet, dass** ein Schrumpfschlauch um den Bewegungsabschnitt (56) des Außenschlauchs (10) und/oder um den Bewegungsabschnitt (59) des Mittelschlauchs (8) verlaufend aufgeschrumpft ist, wobei das Spiralelement (57, 58) zwischen dem Schrumpfschlauch und dem Bewegungsabschnitt (56) des Außenschlauchs (10) und/oder dem Bewegungsabschnitt (59) des Mittelschlauchs (8) angeordnet ist.

7. System nach einem der Ansprüche 3 bis 6, **dadurch gekennzeichnet, dass** ein erstes Rückhaltemittel (62) und ein zweites Rückhaltemittel (64) vorgesehen sind, wobei das erste Rückhaltemittel (62) mit dem Bewegungsabschnitt (56) des Außenschlauchs (10) zusammenwirkt, wobei das zweite Rückhaltemittel (64) mit dem Bewegungsabschnitt (59) des Mittelschlauchs (8) zusammenwirkt, wobei diejenige Rückhaltekraft, die das erste Rückhaltemittel (62) auf den Bewegungsabschnitt (56) des Außenschlauchs (10) ausübt in eine Schiebelementbewegungsrichtung (28, 30) größer ist als diejenige Mitnahmekraft, die das erste Mitnahmemittel (36) auf den Bewegungsabschnitt (56) des Außenschlauchs (10) ausübt, und/oder wobei diejenige Rückhaltekraft, die das zweite Rückhaltemittel (64) auf den Bewegungsabschnitt (59) des Mittelschlauchs (8) ausübt in eine Schiebelementbewegungsrichtung (28, 30) größer ist als diejenige Mitnahmekraft, die das zweite Mitnahmemittel (38) auf den Bewegungsabschnitt (59) des Mittelschlauchs (8) ausübt.

8. System nach Anspruch 7, **dadurch gekennzeichnet, dass** ein erstes Anlagemittel (66) für das erste Rückhaltemittel (62) und ein zweites Anlagemittel (72) für das zweite Rückhaltemittel (64) vorgesehen sind, sodass das erste Rückhaltemittel (62) in die Außenschlauchmitnahmerichtung elastisch nachgiebiger ist als entgegen der Außenschlauchmitnahmerichtung, und sodass das zweite Rückhaltemittel (64) in die Mittelschlauchmitnahmerichtung elastisch nachgiebiger ist als entgegen der Mittelschlauchmitnahmerichtung.

9. System nach Anspruch 7 oder 8, **dadurch gekennzeichnet, dass** das erste Anlagemittel (66) zur Umkehrung der Außenschlauchmitnahmerichtung eine erste Anlagerippe (68) und eine zweite Anlagerippe (70) für das erste Rückhaltemittel (62) aufweist und dass das zweite Anlagemittel (72) zur Umkehrung der Mittelschlauchmitnahmerichtung eine erste Anlagerippe (74) und eine zweite Anlagerippe (76) für das zweite Rückhaltemittel (64) aufweist, wobei das erste Rückhaltemittel (62) und das zweite Rückhaltemittel (64) in einer ersten Schaltstellung jeweils an einer der beiden Anlagerippen (68, 70; 74, 76) anliegen, wobei das erste Rückhaltemittel (62) und das zweite Rückhaltemittel (64) in der zweiten Schaltstellung jeweils an der anderen der beiden Anlagerippen(68, 70; 74, 76) anliegen.

10. System nach einem der Ansprüche 3 bis 9, **dadurch gekennzeichnet, dass** das Schiebelement (26) am ein erstes Haltemittel (32) und/oder ein zweites Haltemittel (34) angeordnet ist bzw. sind, wobei am ersten Haltemittel (32) das erste Mitnahmemittel (36) angeordnet ist, und wobei am zweiten Haltemittel (34) das zweite Mitnahmemittel (38) angeordnet ist, wobei das Schiebeelement (26) ein Paar Mitnehmerrippen (82, 84) aufweist, und wobei das erste Haltemittel (32) oder das zweite Haltemittel (34) zwischen den Mitnehmerrippen (82, 84) axial hin und her bewegbar angeordnet ist.

11. System nach einem der Ansprüche 3 bis 9, **dadurch gekennzeichnet, dass** am Schiebelement (26) ein erstes Haltemittel (32) und/oder ein zweites Haltemittel (34) axial bewegbar angeordnet ist bzw. sind, wobei am ersten Haltemittel (32) das erste Mitnahmemittel (36) angeordnet ist, und wobei am zweiten Haltemittel (34) das zweite Mitnahmemittel (38) angeordnet ist, wobei das erste Haltemittel (32) oder das zweite Haltemittel (34) ein Paar Mitnehmerrippen (132, 134) aufweist, wobei das Schiebeelement ein Haltemitteleingriffelement (130) aufweist, und wobei das Haltemitteleingriffelement (130), und damit das Schiebelement (26), zwischen den Mitnehmerrippen (132, 134) axial hin und her bewegbar ist.

12. System nach Anspruch 8, **dadurch gekennzeichnet, dass** das erste Rückhaltemittel (62) und das zweite Rückhaltemittel (64) jeweils einen ersten Rückhaltemittelabschnitt (94, 96) und einen zweiten Rückhaltemittelabschnitt (98, 100) aufweisen, wobei der erste Rückhaltemittelabschnitt (94, 96) und der zweite Rückhaltemittelabschnitt (98, 100) am ersten Anlagemittel bzw. zweiten Anlagemittel verlagerbar anliegen, wobei in einer ersten Schaltstellung jeweils einer der beiden Rückhaltemittelabschnitte (94, 96; 98, 100) des ersten Rückhaltemittels (62) bzw. des zweiten Rückhaltemittels (64) mit dem Bewegungsabschnitt (56) des Außenschlauchs (10) bzw. dem Bewegungsabschnitt (59) des Mittelschlauchs (8) in Eingriff ist und in einer zweiten Schaltstellung jeweils der andere der beiden Rückhaltemittelabschnitte(94, 96; 98, 100) des ersten Rückhaltemittels (62) bzw. des zweiten Rückhaltemittels (64) mit dem Bewegungsabschnitt (56) des Außenschlauchs (10) bzw. dem Bewegungsabschnitt (59) des Mittelschlauchs (8) in Eingriff ist.

13. System nach einem der vorhergehenden Ansprüche, **dadurch gekennzeichnet, dass** der Innenschlauch (6) an seinem proximalen Ende einen Spülanschluss (54) aufweist und dass das distale Ende des Innenschlauchs (6) mit dem distalen Ende des Außenschlauchs (10) derart fluidverbindbar ist, dass Fluid durch den Innenschlauch (6) dem distalen Ende des Katheter (4) zuführbar ist und zwischen dem Außenschlauch (10) und dem Mittelschlauch (8) und/oder dem Mittelschlauch (8) und dem Innenschlauch (6) aus dem Katheter (4) abführbar ist.

14. System nach einem der Ansprüche 4 bis 13, **dadurch gekennzeichnet, dass** der Katheter (4) zwischen dem ersten Gehäuseabschnitt (44) und dem zweiten Gehäuseabschnitt (50) in einem Führungsschlauch (48) geführt ist.

15. System nach Anspruch 2, **dadurch gekennzeichnet, dass** die Antriebseinheit (20) als Zahnradgetriebe mit einem ersten Zahnrad und einem zweiten Zahnrad ausgebildet ist, wobei das erste Zahnrad mit dem Bewegungsabschnitt (56) des Außenschlauchs (10) zusammenwirkt, und wobei das zweite Zahnrad mit dem Bewegungsabschnitt (59) des Mittelschlauchs (8) zusammenwirkt.

16. System nach einem der vorhergehenden Ansprüche, **dadurch gekennzeichnet, dass** am distalen Endabschnitt (142) des Mittelschlauchs (8) ein Stenteingreifelement (140) angeordnet ist, wobei sich das Stenteingreifelement (140) ausgehend vom distalen Ende des Mittelschlauchs (8) zwischen dem Innenschlauch (6) und dem komprimierten Stent (2) in distaler Richtung (24) erstreckt, wobei das distale Ende (144) des Stenteingreifelements (140) zwischen dem proximalen Ende (12) und dem distalen Ende (14) des komprimierten Stents (2) liegt, und wobei ein distaler Endabschnitt des Stenteingreifelements (140) ein sich quer zur distalen Richtung (22) bzw. proximalen Richtung (24) erstreckendes Eingriffselement (146) aufweist, welches mit dem Stent (2) in Eingriff ist.

17. Katheter (4) für ein System nach einem der vorhergehenden Ansprüche.

18. Antriebseinheit (20) für ein System nach einem der Ansprüche 2 bis 16.

19. Verfahren zum Einführen eines komprimierten, selbstexpandierbaren Stents (2) in insbesondere ein Blutgefäß und zur Freisetzung des Stents (2) in dem insbesondere einen Blutgefäß unter Verwendung eines Systems nach einem Ansprüche 1 bis 16, umfassend die folgenden Schritte:
a) Zuführen des Stents (2) mittels des Katheter (4) an die Bestimmungsposition (16) des distalen Endes (14) des Stents (2);
b) Freisetzen des Stents (2) durch Verschieben des Außenschlauchs (10) in proximaler Richtung (24) und des Mittelschlauchs (8) in distaler Richtung (22), wobei das distale Ende (14) des Stents (2) während der Freisetzung seine Bestimmungsposition (16) beibehält.
